# DEMANDE DE BREVET EUROPEEN

(11) **EP 0 631 088 A1**
(43) Date de publication de la demande: **28.12.1994**
(21) Numéro de dépôt: 94401374.7
(22) Date de dépôt: 17.06.1994
(51) Int. Cl.: F16M 11/04, A61B 6/00

(54) **Agencement de support d'appareil à bras articulé à compensation du poids de la charge**

(30) Priorité: 24.06.1993 FR 9307702
(71) Demandeur: TROPHY RADIOLOGIE, F-94300 Vincennes (FR)
(72) Inventeur: Mesrouze, Michel, F-94500 Champigny (FR); Gaignebet, Etienne, F-94480 Ablons (FR)
(74) Mandataire: Berger, Helmut

(57) **Abrégé**

L'invention concerne un agencement de support d'appareil devant être facilement déplaçable, notamment en translation.

Cet agencement est du type comprenant au moins un bras (1 ou 3) articulé par une extrémité à une structure (2) de support et portant à son autre extrémité une charge (6) au moins représentative dudit appareil et des moyens de stabilisation de celui-ci dans chaque position après un déplacement depuis une position précédente. L'agencement est caractérisé en ce que le bras (1 ou 3) est réalisé sous forme d'un parallélogramme formé par deux barres (8, 9 ; 10, 11) et un dispositif ressort (14, 26) est articulé à une extrémité à une des barres et à son autre extrémité à un dispositif de biellettes (15 ; 25) interposé entre les deux barres de façon que la force du ressort varie d'une façon non linéaire compensant la non-linéarité du couple produit par la charge.

L'invention est utilisable dans le domaine médical par exemple d'une cuve de radiographie dentaire.

## Description

L'invention concerne un agencement de support d'appareil devant être facilement déplaçable, notamment en translation, du type comprenant au moins un bras articulé par une extrémité à une structure de support et portant à son autre extrémité ledit appareil et des moyens de stabilisation de celui-ci dans chaque position après un déplacement depuis une position précédente.

Des agencements de ce type sont déjà connus et il s'est avéré, de façon générale, que la facilité de déplacement de l'appareil porté par le bras s'oppose à la stabilité de la position. Pour néanmoins obtenir que l'appareil reste dans sa nouvelle position qui lui a été assignée, certains agencements prévoient des frottements importants au niveau des articulations, d'autres emploient des contrepoids, ce qui nuit, dans un cas comme dans l'autre, à la souplesse de fonctionnement. On a aussi déjà proposé des agencements de support qui sont du type asservi. Mais ces agencements présentent l'inconvénient qu'ils présentent une structure très complexe.

La présente invention a pour but de proposer un agencement de support assurant un déplacement facile et d'une grande souplesse de l'appareil et qui maintient celui-ci dans la nouvelle position où il a été placé.

Pour atteindre ce but, l'agencement est caractérisé en ce que le bras est réalisé sous forme d'un parallélogramme formé par deux barres et un dispositif ressort est articulé à une extrémité à une des barres et à son autre extrémité à un dispositif de biellettes interposé entre les deux barres de façon que la force du ressort varie d'une façon non linéaire compensant la non-linéarité du couple produit par la charge.

L'invention sera mieux comprise et d'autres buts, caractéristiques détails et avantages de celle-ci apparaîtront plus clairement dans la description explicative qui va suivre faite en référence aux dessins schématiques annexés donnés uniquement à titre d'exemple illustrant un mode de réalisation de l'invention et dans lequel.

La figure 1 est une vue schématique d'un agencement de support à deux bras, selon la présente invention.

La figure 2 est une vue à plus grande échelle du bras intérieur de l'agencement de la figure 1 et montre celui-ci dans deux positions extrêmes.

La figure 3 est une vue à plus grande échelle du bras extérieur de l'agencement selon la figure 1 et montre celui-ci dans deux positions extrêmes.

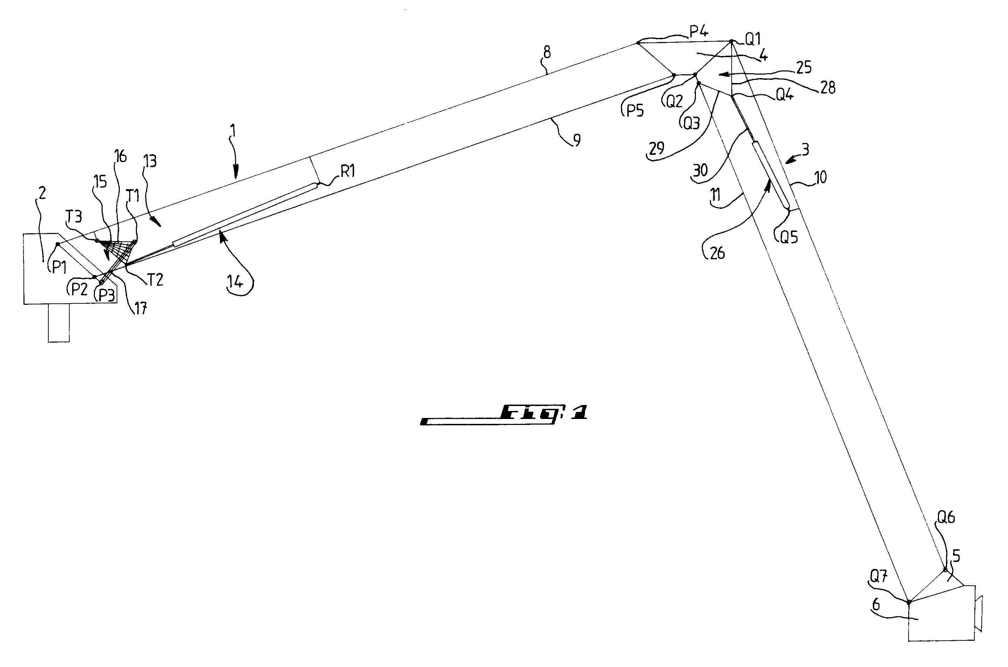

En se référant à la figure 1, on constate qu'un agencement de support d'appareil, par exemple d'une cuve de radiographie dentaire, pendant son utilisation, comprend essentiellement un bras ciseau constitué d'un bras interne 1 articulé à une extrémité à un support fixe ou tournant 2, un bras externe 3 relié par l'intermédiaire d'un coude 4 à l'extrémité libre du bras interne 1 et portant à son autre extrémité, par l'intermédiaire d'un élément formant poignée 5 l'appareil qui doit être facilement déplaçable d'une position à l'autre et resté ensuite dans la position qui lui a été assignée. L'appareil n'est représenté que schématiquement et porte la référence 6. Le support 2 assure le maintien de l'ensemble par rapport à un appui fixe, tel qu'un mur, une table ou autre.

Chaque bras 1 ou 3 est réalisé sous forme d'un parallélogramme déformable dont les deux grands côtés sont formés par des bras respectivement 8, 9 et 10, 11.

Chaque bras comporte un dispositif de stabilisation permettant le déplacement angulaire du bras et assurant le maintien du bras dans chaque position. Chaque dispositif comprend un ressort pouvant être un ressort classique ou un vérin à gaz et un dispositif de biellettes. La longueur des biellettes et leurs points d'articulation sont choisies de telle façon qu'elles permettent de compenser la non-linéarité du couple que l'appareil 6 représente par rapport au coude 4 et au support 2, le ressort présentant une caractéristique force/course sensiblement linéaire.

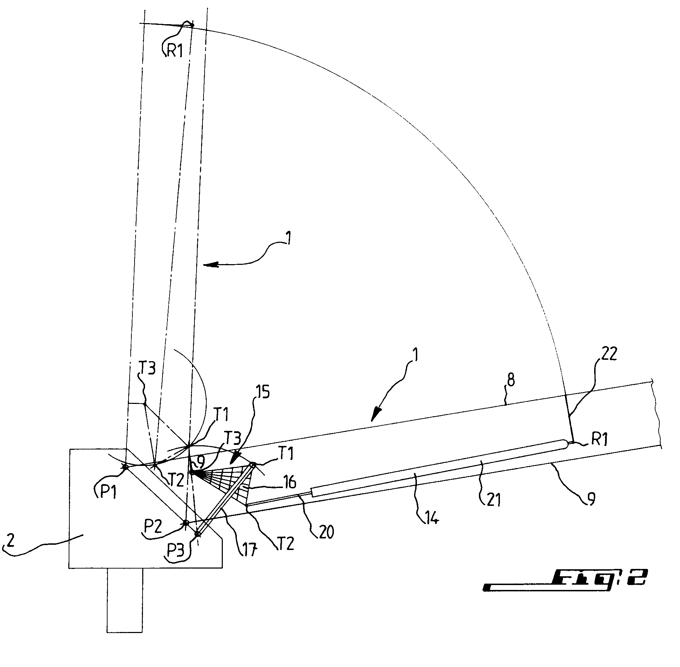

La figure 2 montre à plus grande échelle le bras inférieur 2 et son dispositif de stabilisation c'est-à-dire de compensation. Ce dispositif porte la référence générale 13. Le ressort est indiqué en 14 et le dispositif des biellettes en 15.

Ce dispositif de biellette 15 comprend deux biellettes 16, 17. La biellette 16 présente une forme triangulaire et approximativement isocèle. Les trois sommets portent les références respectivement T1, T2, T3, où T1, T2 constituent les sommets de base. Le sommet T3 est articulé à l'extrémité d'une barrette 9 dont l'autre extrémité est solidaire de la barre 8. La barrette 9 s'étend perpendiculairement à la dernière en direction de l'autre barre 9 et présente une longueur relativement faible, mais prédéterminée selon les considérations qui seront exposées plus loin. Le sommet de base T1, dans la position approximativement horizontale indiquée en traits pleins sur la figure 2 et adjacent à la barre 8 est articulé à l'extrémité de la biellette 17 dont l'autre extrémité est articulée en P3 au support fixe 2. Le point P3 se trouve sur la ligne droite qui relie les points P1, P2 auxquels les deux bras 8 et 9 sont articulés à ce support fixe 2. On constate que le point P3 est situé au côté du point P3, qui est opposé au point d'articulation P1 du bras 8. Le sommet T2 de la biellette triangulaire 16 est articulé à la tige de piston 20 du vérin 14 qui est articulé par l'extrémité de son corps 21 en R1 à une barrette de support 22 fixée à son autre extrémité à la barre 8. La barrette 22 s'étend sensiblement perpendiculairement à la barrette 8 en direction de la barrette 9 et présente une longueur qui est prédéterminée en fonction des considérations qui seront expliquées ci-après. On constate encore que les bras 8 et 9 sont articulés en P4 et P4 au coude 4.

En effet, la forme de la biellette triangulaire 16, la longueur de la biellette 17 et la position des points R1 et T3 sont calculées de telle façon qu'en position basse, c'est-à-dire lorsque les bras 8 et 9 sont pratiquement horizontaux, le ressort 14 travaille en compression et fournit son effort maximal du fait que sa longueur est alors minimale. En effet, grâce à sa direction, le ressort a son efficacité maximale dans cette position horizontale du bras 1 pour contrer les effets de la pesanteur qui a sa valeur maximale dans cette position. Ceci ressort de la figure 2 qui montre le bras 1 dans une position près à la position horizontale. Les dimensions sont d'autre part choisies de façon qu'en position verticale du bras 1 le ressort n'agisse plus sur l'équilibre du bras. Dans cette position les points R1, T2 et T3 sont presque alignés ce qui neutralise l'action du ressort, même s'il est encore bandé. Sur la figure 2 le bras est représenté en traits interrompus dans une position proche de ladite position verticale.

Il s'est avéré en outre que, tout en respectant les conditions qui viennent d'être énoncées, il est possible, par un choix approprié des longueurs ou distances P3-T1 ; P2-P3 ; T1-T2 ; T2-T3 et T3-T1 et en choisissant de façon appropriée les caractéristiques du ressort, d'obtenir que la force du ressort compense le poids agissant sur l'extrémité libre du bras 1 pour toutes les positions intermédiaires de celui-ci.

Le bras extérieur 3 dont les barres 10 et 11 sont articulées au coude 4 respectivement en Q1 et Q2 comporte également un dispositif de biellettes portant la référence générale 25 et sur lequel agit un ressort réalisé sous forme d'un vérin à gaz 26. Le dispositif de biellettes 25 se compose des deux biellettes 28, 29 qui sont articulées l'une à l'autre en Q4 par l'une de leurs extrémités. L'autre extrémité de la biellette 28 est articulée en Q1 à la barre 10 tandis que l'autre extrémité de la biellette 29 est articulée en Q3 à la barre 11. Le point d'articulation Q3 se trouve à une distance relativement faible du point d'articulation Q2. Au point commun Q4 des deux biellettes est reliée la tige de piston du vérin 26 qui est articulée par l'extrémité de son corps en R2 à l'extrémité d'une barrette 32 dont l'autre extrémité est fixée à la barre 10 et qui s'étend perpendiculairement à cette dernière en direction de la barre 11.

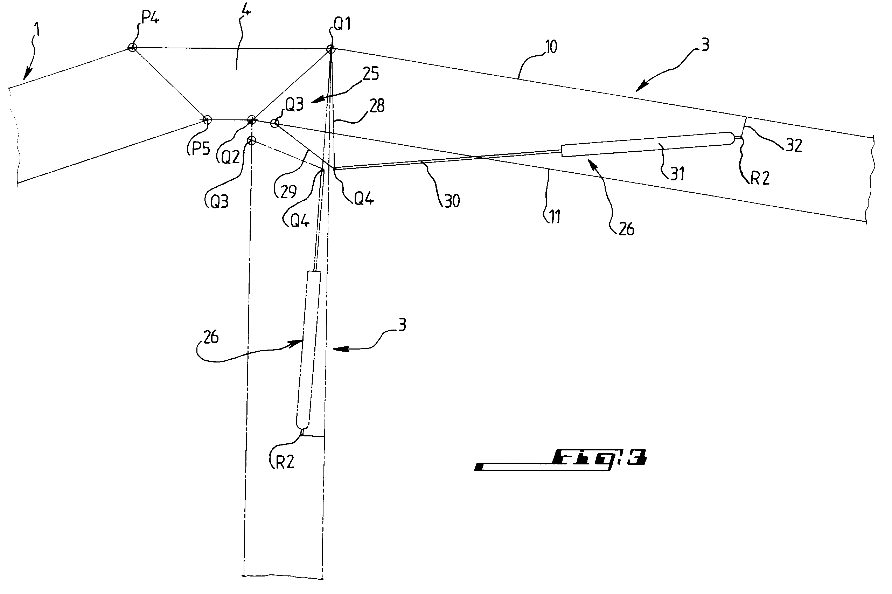

Comme dans le cas du bras intérieur 1, les longueurs des biellettes 28, 29, leurs points d'articulation et les caractéristiques du vérin à gaz sont choisis de façon que dans chaque position angulaire du bras l'effet du poids de la charge 6 soit compensé. Le principe du choix des valeurs de dimensionnement est illustré sur la figure 3 qui représente le bras 1 dans deux positions spécifiques, à savoir une position presque horizontale (en traits continus) et une position verticale (en traits interrompus). Dans la position pratiquement horizontale dans laquelle l'effet de la pesanteur de la charge est maximal, les biellettes 28, 29 assurent une forte compression du ressort formée par le vérin à gaz 26. C'est dans cette position que le ressort produit son effet maximal d'opposition à une déformation du parallélogramme constitué par les deux barres 10 et 11. La position verticale du bras 3 est caractérisée par le fait que la biellette 28 et le vérin 26 sont alignés, ce qui annule l'action du ressort.

Il ressort de la description qui précède, du dispositif qui est représenté aux figures, que l'invention est basée sur le fait qu'il est possible d'obtenir un équilibre permanent du dispositif, dans chaque position angulaire, d'une manière très simple, en utilisant des dispositifs de biellettes articulés à un ressort et en choisissant les valeurs et les points d'articulation ainsi que les caractéristiques du ressort d'une manière appropriée. A titre d'exemple, on obtient cet effet d'équilibre permanent en choisissant les valeurs suivantes : 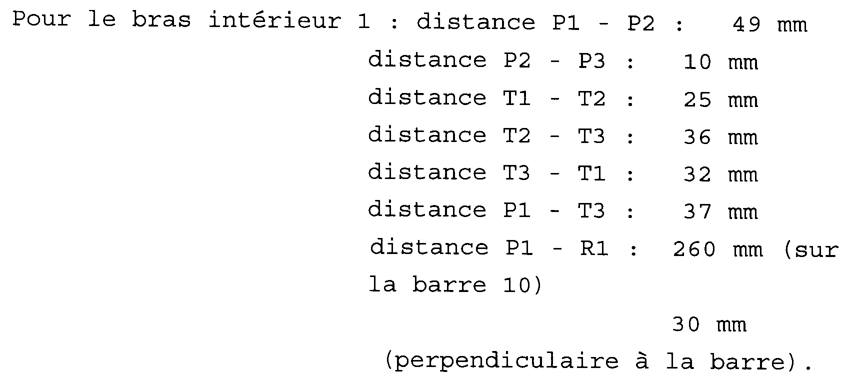
- Pour le bras extérieur 3 :: distance Q1 - Q2 : 49 mm
distance Q2 - Q3 : 10 mm
distance Q1 - Q4 : 59 mm
distance Q3 - Q4 : 33 mm
distance Q1 - Q5 : 278 mm.

Pour tenir compte de la charge et du battement des bras, on choisira en conséquence le ressort, sans qu'il soit nécessaire de modifier les valeurs concernant la longueur et les articulations des biellettes. Si pour une charge et un battement de bras prédéterminés, le ressort à retenir ne correspond à un ressort disponible dans le commerce, il suffit d'un calcul d'adaptation desdites valeurs pour pouvoir utiliser un ressort disponible, tout en conservant approximativement les proportions des valeurs préalablement établies.

## Revendications

1. Agencement de support d'appareil devant être facilement déplaçable, notamment en translation, du type comprenant au moins un bras articulé par une extrémité à une structure de support et portant à son autre extrémité une charge au moins représentative dudit appareil et des moyens de stabilisation de celui-ci dans chaque position après un déplacement depuis une position précédente, caractérisé en ce que le bras (1 ou 3) est réalisé sous forme d'un parallélogramme formé par deux barres (8, 9 ; 10, 11) et un dispositif ressort (14, 26) est articulé à une extrémité à une des barres et à son autre extrémité à un dispositif de biellettes (15 ; 25) interposé entre les deux barres de façon que la force du ressort varie d'une façon non linéaire compensant la non-linéarité du couple produit par la charge.

2. Agencement de support selon la revendication 1, caractérisé en ce que le dispositif de biellettes (15, 25) comprend deux biellettes (16, 17 ; 28, 29) qui sont articulées l'une à l'autre par l'une de leurs extrémités et à l'extrémité libre du dispositif ressort (14, 26), tandis que les autres extrémités des deux biellettes sont articulées respectivement à au moins un bras et un point de la structure de support et en ce que les longueurs des biellettes et les endroits de leur articulation sont choisis de façon que la force de ressort compense dans chaque position du bras le couple produit par le poids de l'appareil, le ressort étant sollicité pour fonctionner en compression.

3. Agencement de support selon la revendication principale, caractérisé en ce que, pour un bras déplaçable entre une position sensiblement verticale à orientation vers le haut et une position sensiblement horizontale, une biellette (16) du dispositif de biellettes (15) présente la forme d'un triangle avantageusement isocèle dont les sommets (T3, T1, T2) sont articulés respectivement (en T3) à la barre supérieure (8), à l'extrémité (en T1) de l'autre biellette (17) et en (T2) au dispositif ressort (14), que la biellette (17) est articulée par son extrémité libre (en P3) au support (2), que la configuration et la longueur des biellettes ainsi que leur articulation sont choisies de façon que le dispositif ressort subit sa compression maximale lorsque le bras (1) se trouve dans sa position sensiblement horizontale et que le point d'articulation (T2) de la biellette (16) au dispositif ressort (14), le point d'articulation (T3) de cette biellette (16) à la barre (8) et le point d'articulation (R1) du dispositif ressort (14) à cette barre (8) se trouve sensiblement sur une même ligne sensiblement verticale, le dispositif ressort (14) subissant alors une compression minimale.

4. Agencement de support selon la revendication 3, caractérisé en ce que le point d'articulation (P3) de la biellette (17) au support (2) se trouve sur la ligne droite reliant les points d'articulation (P1, P2) au support (2), des deux barres (8, 9) du bras (1), le point d'articulation (P3) se trouvant du côté du point d'articulation (P2) qui est opposé à celle du point d'articulation (P1).

5. Agencement de support selon l'une des revendications 1 ou 2, caractérisé en ce que, pour un bras (3) monté pivotant entre une position sensiblement horizontale et une position sensiblement verticale à orientation vers le bas, le dispositif de biellette (25) comprend deux biellettes (28, 29) qui sont reliées par une de leurs extrémités (en Q4) au dispositif ressort (26) et dont les autres extrémités sont articulées respectivement (en Q1 et Q3) aux deux barres (10, 11) du bras pivotant, la longueur des biellettes (28, 29) et leurs articulations étant choisies de façon que le dispositif ressort (26) subisse sa compression maximale dans la position horizontale du bras et sa compression minimale dans la position verticale du bras.

6. Agencement de support selon l'une des revendications précédentes, caractérisé en ce qu'il comprend deux bras (1, 3) en forme de parallélogramme, qui sont articulés l'un à l'autre par l'intermédiaire d'un élément formant coude (4), l'un (1) des deux bras étant articulé au support (2) et l'autre (3) portant l'appareil précité (6).

7. Agencement de support selon l'une des revendications précédentes, caractérisé en ce que le dispositif ressort (14 ; 26) est un vérin à gaz.
